# EUROPEAN PATENT SPECIFICATION

(11) **EP 4 119 111 B1**
(45) Date of publication and mention of the grant of the patent: **04.09.2024**
(21) Application number: 21184976.5
(22) Date of filing: 12.07.2021
(51) Int. Cl.: A61F 13/534, A61F 13/535, A61F 13/537

(54) **A DUAL-CORE ABSORBENT SANITARY ARTICLE AND A METHOD FOR PRODUCING THE SAME**
ABSORBIERENDER HYGIENEARTIKEL MIT ZWEI KERNEN UND VERFAHREN ZUR HERSTELLUNG DAVON
ARTICLE SANITAIRE ABSORBANT À DOUBLE NOYAU ET SON PROCÉDÉ DE PRODUCTION

(43) Date of publication of application: 18.01.2023
(73) Proprietor: Fameccanica.Data S.p.A., 66020 San Giovanni Teatino (CH) (IT)
(72) Inventor: SABLONE, Gabriele, 66020 San Giovanni Teatino (Chieti) (IT); BONELLI, Guido, 66020 San Giovanni Teatino (Chieti) (IT)
(74) Representative: Marchitelli, Mauro

(56) References cited:
- EP-A1- 3 153 141
- EP-A1- 3 284 449
- EP-A1- 3 527 183
- WO-A1-2020/131961

## Description

### Field of the invention

The present invention relates to absorbent sanitary articles, such as disposable diapers, incontinent articles, and the like, having a dual-core absorbent structure.

The present invention also relates to a method for producing such absorbent sanitary articles.

### Prior art

Absorbent articles such as disposable diapers, adult incontinent pads, and the like are manufactured in many different sizes and shapes. Incontinent pads are disposable absorbent articles worn by incontinent persons between the legs and fastened around the waist of the wearer.

An absorbent sanitary article typically has a structure that comprises a central body or chassis having a front section and a rear section. The chassis normally includes a permeable topsheet intended to come into contact with the user's skin when the article is worn, an impermeable backsheet and an absorbent core sandwiched between the topsheet and the backsheet. The front and rear sections of the chassis are normally closed around the user's waist by means of hook-and-loop fasteners, better known as Velcro^{©} fastening devices.

There is a constant effort to make such absorbent articles more comfortable and discrete and less obtrusive for the wearer. This is particularly true in the case of adult incontinent pads. Adult incontinent pads, by their nature, are quite thick so they can handle the relatively large quantities of urine they receive.

The desire for less bulky incontinent pads is particularly great among incontinent persons, many of whom are elderly adults, because it can be embarrassing to wear diaper-like articles. In addition, most individuals would rather use some type of article that could be worn with the undergarments they normally wear.

One key component of an absorbent article that contributes to its size and bulk is the absorbent structure used therein.

The absorbent core of the absorbent sanitary article ideally should be such that the absorbent article absorbs liquid exudates immediately when they are discharged so that such exudates will not pool or otherwise remain on top of the core.

The absorbent core ideally should also be constructed so exudates initially absorbed will be immediately transported to a place within the absorbent core where they can be stored.

The absorbent core should also provide a system of distribution and storage for exudates that efficiently uses the entire capacity of the absorbent core. One problem that often arises in absorbent articles without such a system (particularly those absorbent articles that use the same layer or batt of material to serve the different functions of acquiring, distributing, and storing exudates) is that the storage capacity of the absorbent article is exhausted prematurely. This can occur in several different ways. In many absorbent articles, the saturation of the absorbent material in the region where exudates are initially deposited reduces the ability of the absorbent material to transport additional exudates to other parts of the core. This phenomenon can also lead to the undesirable pooling of exudates on top of the core discussed above.

EP 3 527 183 A1 discloses an absorbent core wherein a first core layer comprises a channel having a first shape and a second core layer comprises a further channel having a second shape and wherein the first and second core layers are disposed one on top of the other such that a third channel shape is formed along the core length and width.

EP 3 153 141 A1 discloses a method for producing an absorbent structure, comprising advancing a first non-woven layer, distributing superabsorbent granular material on the first non-woven layer, volumizing said first non-woven layer by means of a toothed portion that penetrates into the first non-woven layer.

WO 2020/131961 A1 disclose an absorbent core including a first absorbent core construction having multiple spaced-apart sections of a fibrous construction having a fibre structure. A first non-woven sheet is positioned above the fibrous construction. A second nonwoven sheet is positioned below the fibrous construction. The first non-woven sheet is coupled with the second non-woven sheet at locations between adjacent sections of the multiple spaced-apart sections of the fibrous construction. Absorbent material is disposed within the fibre structure, between the first and second non-woven sheets.

EP 3 284 449 A1 discloses an absorbent core comprising at least one absorbent structure comprising a substrate layer and an absorbent layer with channels. The channels are formed by bonding of the first substrate layer to a second substrate layer through the channels so that the channels are at least partially maintained both in the dry state and in the wet state.

### Object and summary of the invention

The object of the present invention is to provide an absorbent sanitary article with an improved shape that has an absorbent structure that quickly acquires and distributes exudates throughout the absorbent core where they can be stored.

An object of the present invention is to provide an absorbent article, such as an adult incontinent pad, having a rapid acquiring, multiple layer absorbent structure having an improved shape that can be worn in the wearer's usual undergarments.

Another object of the present invention is to provide an absorbent structure for an absorbent sanitary article which is especially efficient in acquiring, distributing, and storing liquid exudates as they are deposited on the absorbent article.

In particular, an object of the present invention is to provide an absorbent structure in which the functions of acquiring and distributing exudates are handled by layers that are separate from the layers used for storing exudates.

According to the present invention, these objects are achieved by an absorbent sanitary article having the features of claim 1.

According to another aspect, the invention relates to a method for manufacturing absorbent sanitary articles having the features of claim 13.

The claims form an integral part of the technical disclosure provided here in relation to the invention.

### Brief description of the drawings

The present invention will now be described with reference to the attached drawings, provided purely by way of non-limiting example, wherein:
- Figure 1 is a schematic plan view showing an absorbent sanitary article according to the present invention in an extended position,
- Figure 2 is a schematic cross-section taken along the line II-II of figure 1, and
- Figure 3 is a schematic side view of an embodiment of a machine for manufacturing absorbent sanitary articles according to the present invention.

It should be appreciated that the attached drawings are schematic and not to scale with respect to real products. Various figures may not be represented in the same scale. Also, in various figures some elements may not be shown to better show other elements.

### Detailed description

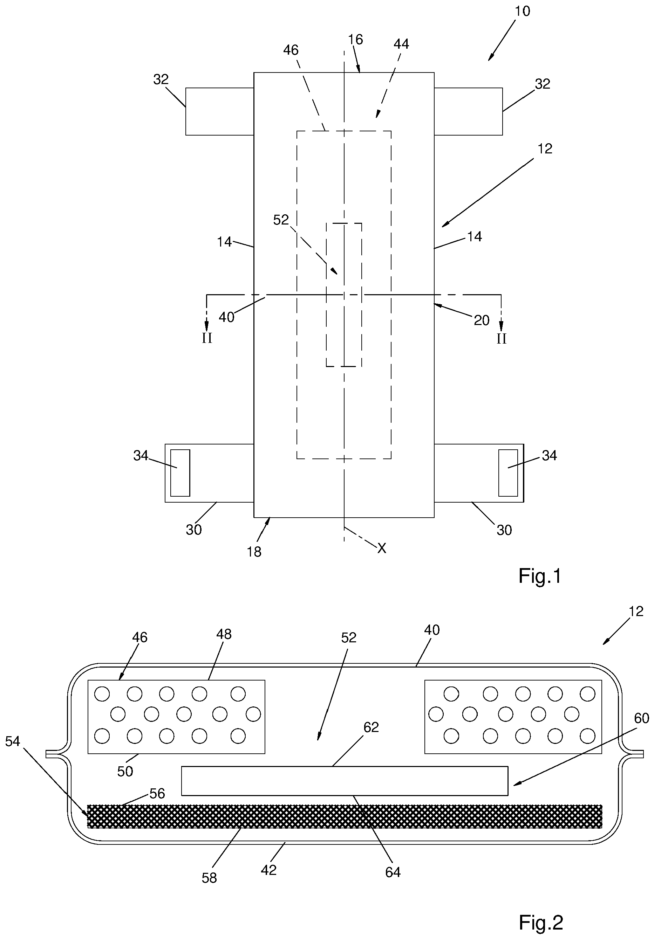

With reference to Figure 1, numeral 10 indicates an absorbent sanitary article according to an embodiment of the present invention.

The absorbent sanitary article 10 comprises a chassis 12 elongated along a longitudinal axis X. The chassis 12 has two side edges 14, a front section 16 and a rear section 18. The front section 16 and the rear section 18 in use are closed around the user's waist. A crotch section 20 extends between the front section 16 and the rear section 18. In use, the crotch section 20 is arranged between the legs of the user.

The absorbent sanitary article 10 comprises a fastening device configured for fastening the front section 16 and the rear section 18 to each other. The fastening device may comprise two back side panels 30 attached to the rear section 18 of the chassis 12 and two front side panels 32 attached to the front section 16, extending laterally beyond respective side edges 14. The back side panels 30 and/or the front side panels 32 may be elastically stretchable in a transverse direction. The back side panels 30 may have distal portions provided with micro-hook elements 34 which form a releasable surface connection with a micro-loop layer provided on the front side panels 32.

With reference to figure 2, the chassis 12 comprises a topsheet 40 made of a permeable material which, in use, is in contact with the user's skin, an impermeable backsheet 42 which, in use, is in contact with the user's undergarments, and an absorbent structure 44 arranged between the topsheet 40 and the backsheet 42. The topsheet 40 and the backsheet 42 are joined to each other, e.g. by welding or by glue, around the absorbent structure 44 along the side edges 14 and transverse edges of the chassis 12.

The absorbent structure 44 comprises a fluff absorbent core 46 adjacent to the topsheet 40 and comprising a mix of cellulose fluff and superabsorbent granular material dispersed in the cellulose fluff.

The fluff absorbent core 46 is a soft pad having a generally flat shape elongated in the longitudinal direction X with two opposite first and second surfaces 48, 50. The first surface 48 may be in contact with the topsheet 40. The fluff absorbent core 46 has at least one through aperture 52 which extends through the mix of cellulose fluff and superabsorbent granular material between the two opposite surfaces 48, 50. In a possible embodiment the through aperture 52 is shaped as a channel (see figure 1) elongated along the longitudinal axis X. The fluff absorbent core 46 may be enclosed in a thin permeable non-woven layer which contains the mix of cellulose fluff and superabsorbent granular material.

The absorbent structure 44 comprises a fluff-free absorbent core 54 adjacent to the backsheet 42. The fluff-free absorbent core 54 comprises a plate-shaped non-woven web containing high quantity of superabsorbent granular material dispersed in the non-woven web.

The fluff-free absorbent core 54 has two opposite third and fourth surfaces 56, 58, generally parallel to the first and second surfaces 48, 50 of the fluff absorbent core 46. The fourth surface 58 may be in contact with the backsheet 42.

The fluff-free absorbent core 54 has a thickness which is considerably less than the thickness of the fluff absorbent core 46. In a possible embodiment, the thickness of the fluff-free absorbent core 54 is comprised between 1 and 4 mm, preferably between 1 and 2 mm.

The fluff-free absorbent core 54 comprises a non-woven material formed by processes such as spunbonding, meltblowing, carding, and the like. Non-limiting examples of suitable fibers include spunbond, spunlaid, meltblown, spunmelt, solvent-spun, electrospun, carded, film fibrillated, melt-film fibrillated, air-laid, dry-laid, wet-laid staple fibers, and other non-woven web materials formed in part or in whole of polymer fibres as known in the art, and workable combinations thereof. Nonwovens do not have a woven or knitted filament pattern. It is to be appreciated that non-wovens having various basis weights can be used. For example, some nonwovens may have a basis weight from about 5 gsm to about 50 gsm, preferably between 8 and 30 gsm.

In a possible embodiment, the fluff-free absorbent core 54 may comprise at least one layer of high loft non-woven material. An high loft non-woven is an ATB (air through bond) in two-component fibres, with high apparent thickness and large gaps between the fibers to accommodate the SAP. The weight range of high loft non-woven webs is comprised between 20 to 150 gsm, preferably between 30 to 80 gsm.

In a possible embodiment, the fluff-free absorbent core 54 comprises super-absorbent granular material dispersed in the non-woven fibres in an amount comprised between 100 to 800 gsm, preferably between 200 and 600 gsm. The fluff-free absorbent core 54 may be manufactured as disclosed in IT102020000019960 by the same Applicant.

In a possible embodiment, the fluff-free absorbent core 54 may be enclosed in a liquid permeable wrapping layer. In a possible embodiment, the fluff-free absorbent core 54 may comprise upper and lower non-woven layers. In a possible embodiment, both upper and lower non-woven layers contain super-absorbent granular material. In a possible embodiment, only the lower non-woven layer contains super-absorbent granular material. In both cases, the fluff-free absorbent core 54 may be enclosed in a liquid permeable wrapping layer.

The absorbent structure 44 comprises an acquisition and diffusion layer (ADL) 60 set between the fluff absorbent core 46 and fluff-free absorbent core 54 and overlapping the at least one through aperture 52 of the fluff absorbent core 46. The acquisition and diffusion layer 60 may be formed by a high loft non-woven material, which does not contain neither cellulose fluff nor superabsorbent polymers. The acquisition and diffusion layer 60 has the shape of a thin plate having opposite fifth and sixth surfaces 62, 64 generally parallel to the surfaces 48, 50 and 56, 58 of the fluff absorbent core 46 and fluff-free absorbent core 54. The fifth and sixth surfaces 62, 64 of the acquisition and diffusion layer 60 are in contact, respectively, with the second surface 50 of the fluff absorbent core 46 and with the third surface 56 of the fluff-free absorbent core 54.

The fifth surface 62 of the acquisition and diffusion layer 60 covers at least the portion of the second surface 50 of the fluff absorbent core 46 on which the at least one aperture 52 extends. The sixth surface of the acquisition and diffusion layer 60 is 30% or more of the third surface 56 of the fluff-free absorbent core 54. The acquisition and diffusion layer 60 may have a mono-layer or a multi-layer structure.

Normally a fluff-free absorbent core has liquid acquisition times longer than a fluff absorbent core. In the arrangement according to the invention, the acquisition and diffusion layer 60 acquires rapidly a surge of liquid, such as urine, passing through the through aperture 52 of the fluff absorbent core 46 and serves as a temporary reservoir for the liquid until the fluff-free absorbent core 42 can absorb the liquid. Also, the acquisition and diffusion layer 60 receives the liquid concentrated mainly in the area facing the through aperture 52 and distributes the liquid over a larger surface to facilitate absorption of the liquid by the fluff-free absorbent core 54. The acquisition and diffusion layer 60 forms a spacer between the fluff absorbent core 46 and the fluff-free absorbent core 42 and allows the liquid passing through the aperture 52 to be distributed over most of the surface of the fluff-free absorbent core 42.

Traditional dual-core absorbent sanitary articles have a very high absorbing capacity but are bulky and obtrusive for the wearer.

The absorbent sanitary article according to the present invention retains the same very high absorbing capacity of prior art dual-core absorbent sanitary articles in a structure which is more comfortable and discrete and less obtrusive for the wearer and that can be worn in the wearer's usual undergarments.

The absorbent sanitary article according to the present invention is particularly rapid in acquiring a liquid surge, typical of incontinent adults, and has a multiple layer absorbent structure which is especially efficient in acquiring, distributing, and storing liquid exudates as they are deposited on the absorbent article.

The functions of acquiring and distributing exudates are handled by the acquisition and diffusion layer 60 which is separate from the fluff absorbent core 46 and the fluff-free absorbent core 54 which are used for storing the exudates.

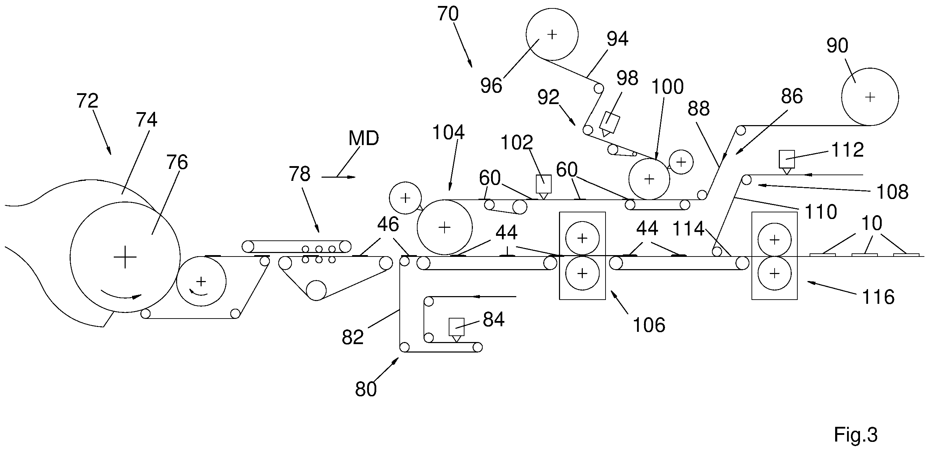

The absorbent sanitary articles 10 according to the present invention may be manufactured by a machine schematically shown in figure 3 and indicated by the reference numeral 70.

The machine 70 comprises a forming unit 72 comprising a stationary forming chamber 74 and a rotating forming wheel 76. The forming chamber 74 contains a mix of cellulose fluff and superabsorbent granular material dispersed in the cellulose fluff. On the outer surface of the forming wheel 76 a plurality of fluff absorbent cores 46 are formed, each comprising a mix of cellulose fluff and superabsorbent granular material and each having at least one through aperture, e.g. a channel shaped through aperture. A compacting conveyor 78 compacts and transports the fluff absorbent cores 46 in a machine direction MD. The fluff absorbent cores 46 are spaced apart from each other in the machine direction MD.

A first web feeding device 80 feeds a continuous topsheet web 82 in the machine direction MD. A first glue dispenser 84 applies a first glue layer on an upper surface of the continuous topsheet web 82.

The individual absorbent cores 46 moving in the machine direction MD are applied on the upper surface of the continuous topsheet web 82 and are attached thereto by the first glue layer.

A second web feeding device 86 feeds a continuous fluff-free absorbent non-woven web 88, comprising superabsorbent granular material dispersed in the non-woven web. The continuous fluff-free absorbent non-woven web 88 may be unwound from a reel 90.

In a possible embodiment, the continuous fluff-free absorbent non-woven web 88 may be manufactured in-line with the machine 70 by a method comprising:
- depositing on a movable surface loose unbound fibres and granular superabsorbent material dispersed in the loose unbound fibres, and
- binding and/or bonding together the loose unbound fibres so as to form an absorbent structure of bound fibres in which granular superabsorbent material is incorporated, where binding involves the use of binders such as glues or similar binding agents and bonding involves only the use of heat, pressure, air, etc. Such method is disclosed in detail in IT102020000019960 by the same Applicant.

A third web feeding device 92 feeds a continuous acquisition and diffusion web 94. The continuous acquisition and diffusion web 94 may be unwound from a reel 96. A second glue dispenser 98 applies a second glue layer on an upper surface of the continuous acquisition and diffusion web 94.

A first cut-and-slip unit 100 transversally cuts the continuous acquisition and diffusion web 94 to form individual acquisition and diffusion layers 60. The individual acquisition and diffusion layers 60 are spaced apart from each other in the direction of movement and are applied on the upper surface of the continuous fluff-free absorbent non-woven web 88 and are attached thereto by the second glue layer.

A third glue dispenser 102 applies a third glue layer on an upper surface of the continuous fluff-free absorbent non-woven web 88.

A second cut-and-slip unit 104 transversally cuts the continuous fluff-free absorbent non-woven web 88 along transverse cutting lines set between each pair of adjacent acquisition and diffusion layers 60, so as to form individual fluff-free absorbent cores 54 each having a respective acquisition and diffusion layer 60 applied thereon. The individual fluff-free absorbent cores 54 are spaced apart from each other in the machine direction MD and are applied over respective fluff absorbent cores 46 attached on the upper surface of the continuous topsheet web 80, as they move in the machine direction MD, so as to form an array of absorbent structures 44 each comprising an acquisition and diffusion layer 60 sandwiched between a respective fluff-free absorbent core 54 and a fluff absorbent core 46. The fluff-free absorbent cores 54 are attached to the respective fluff absorbent cores 46 by the third glue layer.

A compression unit 106 compresses the array of absorbent structures 44. The compression unit 106 may carry out an embossing of the continuous topsheet web 82 and press portions of the continuous topsheet web 82 inside the through apertures 52 of the fluff absorbent cores 46.

Pairs of side panels 30, 32 may be attached to the continuous topsheet web 82.

A fourth web feeding device 108 feeds a continuous backsheet web 110. A fourth glue dispenser 112 applies a fourth glue layer on an upper surface of the continuous backsheet web 110. The continuous backsheet web 110 is then overlapped to the continuous topsheet web 82. The array of absorbent structures 44 is sandwiched between the continuous topsheet web 82 and the continuous backsheet web 110, so as to form a continuous chassis chain 114.

A cutting unit 116 cuts the continuous chassis chain 114 so as to form individual absorbent sanitary articles 10.

A method for producing absorbent sanitary articles according to the present invention comprises:
- providing a continuous topsheet web 82,
- forming a plurality of fluff absorbent cores 46 comprising a mix of cellulose fluff and superabsorbent granular material dispersed in the cellulose fluff, wherein each of said fluff absorbent cores 46 has at least one through aperture 52,
- applying said fluff absorbent cores 46 on the continuous topsheet web 82 spaced apart from each other in a machine direction MD,
- providing a continuous fluff-free absorbent non-woven web 88 comprising superabsorbent granular material dispersed in the non-woven web,
- providing a continuous acquisition and diffusion web 94,
- transversely cutting the continuous acquisition and diffusion web 94 to form individual acquisition and diffusion layers 60,
- spacing apart from each other in a longitudinal direction said individual acquisition and diffusion layers 60,
- applying said individual acquisition and diffusion layers 60 on the continuous fluff-free absorbent non-woven web 88,
- transversely cutting the continuous fluff-free absorbent non-woven web 88 so as to form individual fluff-free absorbent cores 54 each having a respective individual acquisition and diffusion layer 60 applied thereon,
- spacing apart from each other in a longitudinal direction said individual fluff-free absorbent cores 54,
- overlapping said individual fluff-free absorbent cores 54 to respective fluff absorbent cores 46 so as to form an array of absorbent structures 44 each comprising an acquisition and diffusion layer 60 sandwiched between a respective fluff-free absorbent core 54 and a fluff absorbent core 46, wherein the acquisition and diffusion layer 60 overlaps said at least one through aperture 52,
- providing a continuous backsheet web 110,
- applying the continuous backsheet web 110 over the continuous topsheet web 82 and sandwiching said array of absorbent structures 44 between said continuous topsheet web 82 and continuous backsheet web 110, so as to form a chassis chain 114, and
- cutting said chassis chain 114 so as to form individual absorbent sanitary articles 10.

Of course, without prejudice to the principle of the invention, the details of construction and the embodiments can be varied, even significantly, with respect to those illustrated here without departing from the scope of the invention as defined by the following claims.

## Claims

1. An absorbent sanitary article comprising a chassis (12) including a liquid permeable topsheet (40), a liquid impermeable backsheet (42), and an absorbent structure (44) arranged between the topsheet (40) and the backsheet (42), wherein the absorbent structure (44) comprises:
- a fluff absorbent core (46) comprising a mix of cellulose fluff and superabsorbent granular material dispersed in the cellulose fluff, the fluff absorbent core (46) being adjacent to the topsheet (40) and having at least one through aperture (52),
- a fluff-free absorbent core (54) adjacent to the backsheet (42), comprising a non-woven web and superabsorbent granular material dispersed in the non-woven web, and
- an acquisition and diffusion layer (60) set between the fluff absorbent core (46) and the fluff-free absorbent core (54) and overlapping said at least one through aperture (52).

2. The absorbent sanitary article of claim 1, wherein said fluff absorbent core (46) has a generally flat shape elongated in a longitudinal direction (X), and wherein said at least one through aperture (52) has the shape of a channel elongated in said longitudinal direction (X).

3. The absorbent sanitary article of claim 1 or 2, wherein said fluff absorbent core (46) has first and second opposite surfaces (48, 50), said fluff-free absorbent core (54) has third and fourth opposite surfaces (56, 58) and said acquisition and diffusion layer (60) has fifth and sixth opposite surfaces (62, 64), wherein the first surface (48) of the fluff absorbent core (46) is in contact with the topsheet (40), the fourth surface (58) of the fluff-free absorbent core (54) is in contact with the backsheet (42), the fifth surface (62) of the acquisition and diffusion layer (60) is in contact with the second surface (50) of the fluff absorbent core (46), and the sixth surface (64) of the acquisition and diffusion layer (60) is in contact with the third surface (56) of the fluff-free absorbent core (54) .

4. The absorbent sanitary article of claim 3, wherein the fifth surface (62) of the acquisition and diffusion layer (60) covers at least the portion of the second surface (50) of the fluff absorbent core (46) on which said at least one aperture (52) extends.

5. The absorbent sanitary article of claim 3, or claim 4, wherein the sixth surface (64) of the acquisition and diffusion layer (60) is 30% or more of the third surface (56) of the fluff-free absorbent core (54) .

6. The absorbent sanitary article of any of the preceding claims, wherein said fluff-free absorbent core (54) comprises super-absorbent granular material dispersed in the non-woven fibres in an amount comprised between 100 to 800 gsm, preferably between 200 to 600 gsm.

7. The absorbent sanitary article of any of the preceding claims, wherein said fluff-free absorbent core (54) comprises at least one layer of high loft non-woven material.

8. The absorbent sanitary article of any of the preceding claims, wherein said the fluff-free absorbent core (54) comprises upper and lower non-woven layers.

9. The absorbent sanitary article of claim 8, wherein both upper and lower non-woven layers contain super-absorbent granular material.

10. The absorbent sanitary article of claim 8, wherein only the lower non-woven layer contains super-absorbent granular material.

11. The absorbent sanitary article of any of the preceding claims, wherein the fluff-free absorbent core (54) is enclosed in a liquid permeable wrapping layer.

12. A method for producing absorbent sanitary articles, comprising:
- providing a continuous topsheet web (82),
- forming a plurality of fluff absorbent cores (46) comprising a mix of cellulose fluff and superabsorbent granular material dispersed in the cellulose fluff, wherein each of said fluff absorbent cores (46) has at least one through aperture (52),
- applying said fluff absorbent cores (46) on the continuous topsheet web (82) spaced apart from each other in a machine direction (MD),
- providing a continuous fluff-free absorbent non-woven web (88) comprising superabsorbent granular material dispersed in the non-woven web,
- providing a continuous acquisition and diffusion web (94),
- transversely cutting the continuous acquisition and diffusion web (94) to form individual acquisition and diffusion layers (60),
- spacing apart from each other in a longitudinal direction said individual acquisition and diffusion layers (60),
- applying said individual acquisition and diffusion layers (60) on the continuous fluff-free absorbent non-woven web (88),
- transversely cutting the continuous fluff-free absorbent non-woven web (88) so as to form individual fluff-free absorbent cores (54) each having a respective individual acquisition and diffusion layer (60) applied thereon,
- spacing apart from each other in a longitudinal direction said individual fluff-free absorbent cores (54),
- overlapping said individual fluff-free absorbent cores (54) to respective fluff absorbent cores (46) so as to form an array of absorbent structures (44) each comprising an acquisition and diffusion layer (60) sandwiched between a respective fluff-free absorbent core (54) and a fluff absorbent core (46), wherein the acquisition and diffusion layer (60) overlaps said at least one through aperture (52),
- providing a continuous backsheet web (110),
- applying the continuous backsheet web (110) over the continuous topsheet web (82) and sandwiching said array of absorbent structures (44) between said continuous topsheet web (82) and continuous backsheet web (110) so as to form a chassis chain (114), and
- cutting said chassis chain (114) so as to form individual absorbent sanitary articles (10).

13. The method of claim 12, comprising compressing said array of absorbent structures (44) and pressing said continuous topsheet web (82) into said through apertures (52) of said fluff absorbent cores (46).

14. The method of claim 12 or claim 13, comprising forming in-line said continuous fluff-free absorbent non-woven web (88) by a method comprising:
- depositing on a movable surface loose unbound fibres and granular superabsorbent material dispersed in said loose unbound fibres, and
- binding and/or bonding together said loose unbound fibres so as to form an absorbent structure of bound fibres in which granular superabsorbent material is incorporated.

## Patentansprüche

1. Absorbierender Hygieneartikel, umfassend ein Gehäuse (12) einschließlich einer flüssigkeitsdurchlässigen Deckschicht (40), einer flüssigkeitsundurchlässigen hinteren Schicht (42) und einer zwischen der Deckschicht (40) und der hinteren Schicht (42) angeordneten absorbierenden Struktur (44), wobei die absorbierende Struktur (44) umfasst:
- einen absorbierenden Flaumkern (46), umfassend eine Mischung von Zelluloseflaum und in dem Zelluloseflaum dispergiertem superabsorbierendem granulatförmigem Material, wobei der absorbierende Flaumkern (46) an die Deckschicht (40) angrenzt und mindestens eine Durchgangsöffnung (52) aufweist,
- einen flaumfreien absorbierenden Kern (54) neben der hinteren Schicht (42), umfassend eine Vliesbahn und ein in der Vliesbahn dispergiertes superabsorbierendes granulatförmiges Material, und
- eine Aufnahme- und Diffusionsschicht (60), die zwischen dem absorbierenden Flaumkern (46) und dem flaumfreien absorbierenden Kern (54) angeordnet ist und die mindestens eine Durchgangsöffnung (52) überlappt.

2. Absorbierender Hygieneartikel nach Anspruch 1, wobei der absorbierende Flaumkern (46) eine im Wesentlichen flache Form aufweist, die in einer Längsrichtung (X) langgestreckt ist, und wobei die mindestens eine Durchgangsöffnung (52) die Form eines in der Längsrichtung (X) langgestreckten Kanals aufweist.

3. Absorbierender Hygieneartikel nach Anspruch 1 oder 2, wobei der absorbierende Flaumkern (46) eine erste und eine zweite gegenüberliegende Oberfläche (48, 50) aufweist, der flaumfreie absorbierende Kern (54) eine dritte und eine vierte gegenüberliegende Oberfläche (56, 58) aufweist, und die Aufnahme- und Diffusionsschicht (60) eine fünfte und eine sechste gegenüberliegende Oberfläche (62, 64) aufweist,
wobei die erste Oberfläche (48) des absorbierenden Flaumkerns (46) mit der Deckschicht (40) in Kontakt steht, die vierte Oberfläche (58) des flaumfreien absorbierenden Kerns (54) mit der hinteren Schicht (42) in Kontakt steht, die fünfte Oberfläche (62) der Aufnahme- und Diffusionsschicht (60) mit der zweiten Oberfläche (50) des absorbierenden Flaumkerns (46) in Kontakt steht,
und die sechste Oberfläche (64) der Aufnahme- und Diffusionsschicht (60) mit der dritten Oberfläche (56) des flaumfreien absorbierenden Kerns (54) in Kontakt steht.

4. Absorbierender Hygieneartikel nach Anspruch 3, wobei die fünfte Oberfläche (62) der Aufnahme- und Diffusionsschicht (60) zumindest den Abschnitt der zweiten Oberfläche (50) des absorbierenden Flaumkerns (46) bedeckt, auf dem sich die mindestens eine Öffnung (52) erstreckt.

5. Absorbierender Hygieneartikel nach Anspruch 3 oder Anspruch 4, wobei die sechste Oberfläche (64) der Aufnahme- und Diffusionsschicht (60) 30 % oder mehr der dritten Oberfläche (56) des flaumfreien absorbierenden Kerns (54) beträgt.

6. Absorbierender Hygieneartikel nach einem der vorstehenden Ansprüche, wobei der flaumfreie absorbierende Kern (54) ein superabsorbierendes granulatförmiges Material umfasst, das in den Vliesfasern in einer Menge zwischen 100 und 800 gsm, vorzugsweise zwischen 200 und 600 gsm, dispergiert ist.

7. Absorbierender Hygieneartikel nach einem der vorstehenden Ansprüche, wobei der flaumfreie absorbierende Kern (54) mindestens eine Schicht aus hochvoluminösem Vliesmaterial umfasst.

8. Absorbierender Hygieneartikel nach einem der vorstehenden Ansprüche, wobei der flaumfreie absorbierende Kern (54) eine obere und eine untere Vliesschicht umfasst.

9. Absorbierender Hygieneartikel nach Anspruch 8, wobei sowohl die obere als auch die untere Vliesschicht superabsorbierendes granulatförmiges Material enthalten.

10. Absorbierender Hygieneartikel nach Anspruch 8, wobei lediglich die untere Vliesschicht superabsorbierendes granulatförmiges Material enthält.

11. Absorbierender Hygieneartikel nach einem der vorstehenden Ansprüche, wobei der flaumfreie absorbierende Kern (54) in einer flüssigkeitsdurchlässigen Hüllschicht umschlossen ist.

12. Verfahren zum Herstellen absorbierender Hygieneartikel, umfassend:
- Bereitstellen einer kontinuierlichen Deckschichtbahn (82),
- Bilden einer Vielzahl von absorbierenden Flaumkernen (46), umfassend eine Mischung von Zelluloseflaum und in dem Zelluloseflaum dispergiertem superabsorbierendem granulatförmigem Material, wobei jeder der absorbierenden Flaumkerne (46) mindestens eine Durchgangsöffnung (52) aufweist,
- Aufbringen der absorbierenden Flaumkerne (46) auf die kontinuierliche Deckschichtbahn (82) in einer Maschinenrichtung (MD) voneinander beabstandet,
- Bereitstellen einer kontinuierlichen flaumfreien absorbierenden Vliesbahn (88), umfassend ein in der Vliesbahn dispergiertes superabsorbierendes granulatförmiges Material,
- Bereitstellen einer kontinuierlichen Aufnahme- und Diffusionsbahn (94),
- Querschneiden der kontinuierlichen Aufnahme- und Diffusionsbahn (94) zum Bilden einzelner Aufnahme- und Diffusionsschichten (60),
- Beabstanden der einzelnen Aufnahme- und Diffusionsschichten (60) in einer Längsrichtung voneinander,
- Aufbringen der einzelnen Aufnahme- und Diffusionsschichten (60) auf die kontinuierliche flaumfreie absorbierende Vliesbahn (88),
- Querschneiden der kontinuierlichen flaumfreien absorbierenden Vliesbahn (88), um einzelne flaumfreie absorbierende Kerne (54) zu bilden, auf denen jeweils eine einzelne Aufnahme- und Diffusionsschicht (60) aufgebracht ist,
- Beabstanden der einzelnen flaumfreien absorbierenden Kerne (54) in einer Längsrichtung voneinander,
- Überlappen der einzelnen flaumfreien absorbierenden Kerne (54) mit jeweiligen absorbierenden Flaumkernen (46), um eine Anordnung von absorbierenden Strukturen (44) zu bilden, die jeweils eine Aufnahme- und Diffusionsschicht (60) umfassen, die zwischen einem jeweiligen flaumfreien absorbierenden Kern (54) und einem absorbierenden Flaumkern (46) eingeschoben ist, wobei die Aufnahme- und Diffusionsschicht (60) die mindestens eine Durchgangsöffnung (52) überlappt,
- Bereitstellen einer kontinuierlichen hinteren Schichtbahn (110),
- Aufbringen der kontinuierlichen hinteren Schichtbahn (110) über die kontinuierliche Deckschichtbahn (82) und Einschieben der Anordnung absorbierender Strukturen (44) zwischen der kontinuierlichen Deckschichtbahn (82) und der kontinuierlichen hinteren Schichtbahn (110), um eine Gehäusekette (114) zu bilden, und
- Schneiden der Gehäusekette (114) um einzelne absorbierende Hygieneartikel (10) zu bilden.

13. Verfahren nach Anspruch 12, umfassend Zusammendrücken der Anordnung absorbierender Strukturen (44) und Einpressen der kontinuierlichen Deckschichtbahn (82) in die Durchgangsöffnungen (52) der absorbierenden Flaumkerne (46).

14. Verfahren nach Anspruch 12 oder Anspruch 13, umfassend Inline-Bilden der kontinuierlichen flaumfreien absorbierenden Vliesbahn (88) durch ein Verfahren, umfassend:
- Ablegen von losen ungebundenen Fasern und von granulatförmigem superabsorbierendem Material, das in den losen ungebundenen Fasern dispergiert ist, auf einer beweglichen Oberfläche und
- Binden und/oder Miteinanderverbinden der losen ungebundenen Fasern, um eine absorbierende Struktur aus gebundenen Fasern zu bilden, in die granulatförmiges superabsorbierendes Material eingearbeitet ist.

## Revendications

1. Article sanitaire absorbant comprenant un châssis (12) incluant une feuille supérieure (40) perméable aux liquides, une feuille arrière (42) imperméable aux liquides, et une structure absorbante (44) agencée entre la feuille supérieure (40) et la feuille arrière (42), dans lequel la structure absorbante (44) comprend :
- un noyau absorbant duveté (46) comprenant un mélange de duvet de cellulose et de matériau granulaire superabsorbant dispersé dans le duvet de cellulose, le noyau absorbant duveté (46) étant adjacent à la feuille supérieure (40) et présentant au moins une ouverture traversante (52),
- un noyau absorbant (54) sans duvet adjacent à la feuille arrière (42), comprenant une bande non tissée et un matériau granulaire superabsorbant dispersé dans la bande non tissée, et
- une couche d'acquisition et de diffusion (60) disposée entre le noyau absorbant duveté (46) et le noyau absorbant (54) sans duvet et chevauchant ladite au moins une ouverture traversante (52).

2. Article sanitaire absorbant selon la revendication 1, dans lequel ledit noyau absorbant duveté (46) présente une forme généralement plate allongée dans une direction longitudinale (X), et dans lequel ladite au moins une ouverture traversante (52) présente la forme d'un canal allongé dans ladite direction longitudinale (X).

3. Article sanitaire absorbant selon la revendication 1 ou la revendication 2, dans lequel ledit noyau absorbant duveté (46) présente des première et deuxième surfaces (48, 50) opposées, ledit noyau absorbant (54) sans duvet présente des troisième et quatrième surfaces (56, 58) opposées et ladite couche d'acquisition et de diffusion (60) présente des cinquième et sixième surfaces (62, 64) opposées, dans lequel la première surface (48) du noyau absorbant duveté (46) est en contact avec la feuille supérieure (40), la quatrième surface (58) du noyau absorbant (54) sans duvet est en contact avec la feuille arrière (42), la cinquième surface (62) de la couche d'acquisition et de diffusion (60) est en contact avec la deuxième surface (50) du noyau absorbant duveté (46), et la sixième surface (64) de la couche d'acquisition et de diffusion (60) est en contact avec la troisième surface (56) du noyau absorbant (54) sans duvet.

4. Article sanitaire absorbant selon la revendication 3, dans lequel la cinquième surface (62) de la couche d'acquisition et de diffusion (60) recouvre au moins la partie de la deuxième surface (50) du noyau absorbant duveté (46) sur laquelle ladite au moins une ouverture (52) s'étend.

5. Article sanitaire absorbant selon la revendication 3, ou la revendication 4, dans lequel la sixième surface (64) de la couche d'acquisition et de diffusion (60) est de 30 % ou plus de la troisième surface (56) du noyau absorbant (54) sans duvet.

6. Article sanitaire absorbant selon l'une quelconque des revendications précédentes, dans lequel ledit noyau absorbant (54) sans duvet comprend un matériau granulaire superabsorbant dispersé dans les fibres non tissées en une quantité comprise entre 100 à 800 grammes par mètre carré, de préférence entre 200 à 600 grammes par mètre carré.

7. Article sanitaire absorbant selon l'une quelconque des revendications précédentes, dans lequel ledit noyau absorbant (54) sans duvet comprend au moins une couche de matériau non tissé à gonflant élevé.

8. Article sanitaire absorbant selon l'une quelconque des revendications précédentes, dans lequel ledit noyau absorbant (54) sans duvet comprend des couches non tissées supérieure et inférieure.

9. Article sanitaire absorbant selon la revendication 8, dans lequel les couches non tissées supérieure et inférieure contiennent toutes deux un matériau granulaire superabsorbant.

10. Article sanitaire absorbant selon la revendication 8, dans lequel seule la couche non tissée inférieure contient un matériau granulaire superabsorbant.

11. Article sanitaire absorbant selon l'une quelconque des revendications précédentes, dans lequel le noyau absorbant (54) sans duvet est renfermé dans une couche d'enveloppement perméable aux liquides.

12. Procédé de production d'articles sanitaires absorbants, comprenant :
- une fourniture d'une bande (82) de feuille supérieure continue,
- une formation d'une pluralité de noyaux absorbants duvetés (46) comprenant un mélange de duvet de cellulose et de matériau granulaire superabsorbant dispersé dans le duvet de cellulose, dans lequel chacun desdits noyaux absorbants duvetés (46) présente au moins une ouverture traversante (52),
- une application desdits noyaux absorbants duvetés (46) sur la bande (82) de feuille supérieure continue espacés les uns des autres dans une direction de machine (MD),
- une fourniture d'une bande non tissée absorbante sans duvet continue (88) comprenant un matériau granulaire superabsorbant dispersé dans la bande non tissée,
- une fourniture d'une bande d'acquisition et de diffusion continue (94),
- une découpe transversale de la bande d'acquisition et de diffusion continue (94) pour former des couches d'acquisition et de diffusion (60) individuelles,
- un espacement les unes des autres dans une direction longitudinale desdites couches d'acquisition et de diffusion (60) individuelles,
- une application desdites couches d'acquisition et de diffusion (60) individuelles sur la bande non tissée absorbante sans duvet continue (88),
- une découpe transversale de la bande non tissée absorbante sans duvet continue (88) de façon à former des noyaux absorbants (54) sans duvet individuels présentant chacun une couche d'acquisition et de diffusion (60) individuelle respective appliquée sur ceux-ci,
- un espacement les unes des autres dans une direction longitudinale desdits noyaux absorbants sans duvet (54) individuels,
- un chevauchement desdits noyaux absorbants (54) sans duvet individuels sur les noyaux absorbants duvetés (46) respectifs de façon à former un réseau de structures absorbantes (44) comprenant chacune une couche d'acquisition et de diffusion (60) prise en sandwich entre un noyau absorbant (54) sans duvet respectif et un noyau absorbant duveté (46), dans lequel la couche d'acquisition et de diffusion (60) chevauche ladite au moins une ouverture traversante (52),
- une fourniture d'une bande (110) de feuille arrière continue,
- une application de la bande (110) de feuille arrière continue sur la bande (82) de feuille supérieure continue et une prise en sandwich dudit réseau de structures absorbantes (44) entre lesdites bande (82) de feuille supérieure continue et bande (110) de feuille arrière continue de façon à former une chaîne (114) de châssis, et
- une découpe de ladite chaîne (114) de châssis de façon à former des articles sanitaires absorbants (10) individuels.

13. Procédé selon la revendication 12, comprenant une compression dudit réseau de structures absorbantes (44) et un pressage de ladite bande (82) de feuille supérieure continue dans lesdites ouvertures traversantes (52) desdits noyaux absorbants duvetés (46).

14. Procédé selon la revendication 12 ou la revendication 13, comprenant une formation en ligne de ladite bande non tissée absorbante sans duvet continue (88) par un procédé comprenant :
- un dépôt sur une surface mobile de fibres non liées libres et d'un matériau superabsorbant granulaire dispersé dans lesdites fibres non liées libres, et
- un liage et/ou un ensimage desdites fibres non liées libres ensemble de façon à former une structure absorbante de fibres liées dans lesquelles un matériau superabsorbant granulaire est incorporé.
